# EUROPEAN PATENT SPECIFICATION

(11) **EP 1 641 824 B1**
(45) Date of publication and mention of the grant of the patent: **21.07.2010**
(21) Application number: 04737019.2
(22) Date of filing: 18.06.2004
(51) Int. Cl.: C07K 14/62, C07K 14/64, C07K 14/65, C07K 14/575, A61K 38/22, A61K 38/28, A61K 38/30, A61P 3/10, A61P 15/00, A61P 15/08, A61P 15/14, A61P 19/00, A61P 35/00

(54) **RELAXIN SUPERFAMILY PEPTIDE ANALOGUES**
ANALOGA VON PEPTIDEN DER RELAXIN-SUPERFAMILIE
ANALOGUES PEPTIDIQUES DE LA SUPERFAMILLE DES RELAXINES

(30) Priority: 20.06.2003 AU 2003903124
(43) Date of publication of application: 05.04.2006
(73) Proprietor: Howard Florey Institute of Experimental Physiology and Medicine, Parkville, VIC 3010 (Victoria) (AU); The University of Melbourne, Parkville, VIC 3010 (AU)
(72) Inventor: DEL BORGO, Mark, Howard Florey Institute, Parkville, Victoria 3010 (AU); WADE, John, D., Howard Florey Institute, Parkville, Victoria 3010 (AU); BATHGATE, Ross D., Howard Florey Institute, Parkville, Victoria 3010 (AU); HUGHES, Richard A., Parkville, Victoria 3010 (AU)
(74) Representative: Walker, Ross Thomson
(86) International application number: PCT/AU2004/000798
(87) International publication number: WO 2004/113381

(56) References cited:
- EP-A- 0 251 615
- WO-A-03/030930
- M P DEL BORGO ET AL.: "Analofgs of insulin-like peptide 3 (INSL3) B-chain are LGR8 antagonists in vitro and in vivo" JOURNAL OF BIOLOGICAL CHEMISTRY., vol. 281, no. 19, 12 May 2006 (2006-05-12), pages 13068-13074, XP002460327 US AMERICAN SOCIETY OF BIOLOGICAL CHEMISTS, BALTIMORE, MD.
- BULLESBACH ERIKA ET AL.: 'Preparation of partially protected des-alanineB30-insulin-B-chain-disulfide and its use in semisynthesis' INTERNATIONAL JOURNAL OF PEPTIDE & PROTEIN RESEARCH vol. 20, no. 3, 1982, pages 207 - 217, XP008063150
- BULLESBACH E. ET AL.: 'Semisynthesis with insulin-B-chain' CHEM. PEPT. PROTEINS, PROC. USSR-FRG SYMP. 3RD vol. 1, 1982, pages 327 - 335, XP008063149
- IVANOV CH. ET AL.: 'Structure elucidation of the thiolated and oxidated B insulin chain by sephadex gel molecular weight determination' DOKLADY BOLGARSKOI AKADEMII NAUK vol. 29, no. 11, 1976, pages 1641 - 1644, XP008063154

## Description

### Field of the Invention

This invention relates to generally to conformationally constrained, monomeric peptide analogues of the relaxin superfamily of heteromeric proteins and in particular, to monomeric cyclic analogues of the B-chain of relaxin superfamily proteins, methods of making these analogues, and uses thereof.

### Background of the Invention

The relaxin superfamily currently comprises 10 members with a relatively high degree of sequence homology. These family members include insulin, insulin-like grown factors I and II, relaxin 1, 2 and 3, and the insulin-like hormones INSL3, 4, 5 and 6. The relaxin superfamily members have a wide range of biological activities which are well described in the art.

The critical action of insulin has been described for decades. Insulin has a key role in cellular metabolism. Many subjects with diabetes require daily administration of insulin.

Relaxin is an insulin-like peptide having two separate chains (A and B) joined by two interchain and one intrachain disulfide bond. The actions of relaxin include an ability to inhibit myometrial contractions, stimulation of remodelling of connective tissue and induction of softening of the tissues of the birth canal. Additionally, relaxin increases growth and differentiation of the mammary gland and nipple and induces the breakdown of collagen, one of the main components of connective tissue. Relaxin decreases collagen synthesis and increases the release of collagenases (Unemori et al (1990) J. Biol. Chem. 265, 10682-10685). These findings were recently confirmed by the establishment of the relaxin gene-knockout mouse (Zhao et al (1999) Endocrinology 140, 445-453), which exhibited a number of phenotypic properties associated with pregnancy. Female mice lacking a functionally active relaxin gene failed to relax and elongate the interpubic ligament of the pubic symphysis and could not suckle their pups, which in turn, died within 24 hours unless cross-fostered to relaxin wild type or relaxin heterozygous foster mothers.

Evidence has accumulated to suggest that relaxin is more than a hormone of pregnancy and acts on cells and tissues other than those of the female reproductive system. Relaxin causes a widening of blood vessels (vasodilatation) in the kidney, mesocaecum, lung and peripheral vasculature, which leads to increased blood flow or perfusion rates in these tissues (Bani et al (1997) Gen. Pharmacol. 28, 13-22). It also stimulates an increase in heart rate and coronary blood flow, and increases both glomerular filtration rate and renal plasma flow (Bani et al (1997) Gen. Pharmacol. 28, 13-22). Relaxin has also been found to inhibit histamine release and the accumulation of calcium, as well as promote nitric oxide synthesis, during cardiac anaphylaxis (Masini et al., Br J Pharmacol. 2002 Oct;137(3):337-44). The brain is another target tissue for relaxin where the peptide has been shown to bind to receptors (Osheroff et al (1991) Proc. Nal. Acad. Sci. U.S.A. 88, 6413-6417; Tan et al (1999) Br. J. Pharmacol 127, 91-98) in the circumventricular organs to affect blood pressure and drinking (Parry et al (1990) J Neuroendocrinol. 2, 53-58; Summerlee et al (1998) Endocrinology 139, 2322-2328; Sinnahay et al (1999) Endocrinology 140, 5082-5086).

Important clinical uses arise for relaxin in various diseases responding to fibrotic breakdown, connective tissue remodelling, scleroderma, vasodilation, such as coronary artery disease, peripheral vascular disease, kidney disease associated with arteriosclerosis or other narrowing of kidney capillaries, or other capillaries narrowing in the body, such as in the eyes or in the peripheral digits, the mesocaecum, lung and peripheral vasculature, and neurological modification. Relaxin has also been implicated in depression of platelet aggregation and their release by megakaryocytes, and may thus be associated with clotting disorders.

The INSL peptides, such as INSL3, may be involved in various physiological actions, including descent of the gonads. In particular, INSL3 has been shown to be involved in maturation and descent of the testes (Ivell & Hartnung, Mol Hum Reprod 2003, 9(4): 175-181), as well as the survival of sperm cells, development of ovarian follicles and maturation of the oocyte (Kawamura et al., 2004, PNAS 101, 7323-7328). Therefore, potential clinical applications of INSL3 agonists and antagonists include the treatment of fertility disorders, or the control of fertility levels.

INSL3 has been implicated in regulation of relaxin activity in the heart (Tan et al., Eur J Pharmacol. 2002 Dec 20;457(2-3):153-60), and may thus play an important regulatory role in cardiovascular disease.

Furthermore, INSL3 polymorphisms have been hyperplastic and neoplastic disorders of the thyroid gland (Hombach-Klonisch et al., Int J Oncol 2003 May; 22(5): 993-1001), suggesting a role for this relaxin superfamily member in the etiology of these pathologies.

The biological actions of the relaxin superfamily members are mediated through hormone receptors. In particular, the insulin receptor, the IGF-I and IGF-II receptors, GPCR135, GPCR142, as well as LGR7 and LGR8. LGR7 is the relaxin receptor, which binds all human relaxin peptides with high affinity, but has very low affinity for INSL3 (Bathgate et al., Trends in Endocrinology and Metabolism 2003, 14: 207-213). LGR8 is the INSL3 receptor, which binds INSL3 with high affinity. Knockout mice which lack a LGR8 gene have identical phenotype to the INSL3 knockout mice. However in the human, LGR8 will also bind relaxin, although it has a higher affinity for INSL3 (Bathgate et al., Trends in Endocrinology and Metabolism 2003, 14: 207-213).

The LGR8 receptor is expressed in the gubernaculum, ovary and testis, where in the latter organ it mediates INSL3's actions on testes descent (Bathgate et al., Trends in Endocrinology and Metabolism, 2003, 14: 207-213). In the ovary, LGR8 mediates follicular development and in both the testes and ovaries, it is implicated in male and female germ-cell maturation (Kawamura et al., 2004, PNAS 101, 7323-7328). LGR8 is also expressed in the kidney, bone, peripheral-blood leucocytes, muscle, brain, thyroid, and uterus (Hsu et al., Science, 295: 671-674). Hence, INSL3 has potential actions on kidney and thyroid function, central actions on the brain, modulation of muscle function, effects on bone and blood cell function and reproductive actions on the uterus.

There is a need for analogues that are relaxin-superfamily analogues which bind at the biological receptors for the relaxin superfamily proteins. Ideally, such analogues would include ligands, such as agonists, reverse agonists, partial agonists, mixed agonists/antagonists and full antagonists, which bind at the relaxin superfamily member receptors and initiate, inhibit, activate, or otherwise control, the biological activities of members of this protein superfamily. Analogues of the relaxin superfamily proteins could provide the opportunity for a wide range of therapeutic interventions.

Some derivatives of relaxin are disclosed is WO-A-030 30930 and EP-A-251615.

### Summary of the Invention

In its broadest form, the present invention relates to peptide analogues of members of the relaxin superfamily of proteins, the analogues having a binding activity at a relaxin protein superfamily receptor, methods of producing the analogues, and pharmaceutical compositions, methods of treatment, and uses of the peptide analogues.

In a first aspect, the analogue is a conformationally constrained, monomeric cyclic peptide analogue of a B-chain of a relaxin superfamily member protein which binds to a biological target of the relaxin, superfamily protein, the protein being selected from IGF-I, IGF-II, relaxin 1, relaxin 2, relaxin 3, INSL3, INSL4, INSL5 or INSL6.

The monomeric peptide analogues of the invention are produced by modification of a turn or loop moiety of the B-chain of the relaxin superfamily protein, the modification involving selection of at least a first and a second amino acid residue with an alpha-helix or beta-strand carbon separation distance of less than six Angstroms and cross-linking the first and second amino acids, wherein the cross-link conformationally constrains the analogue.

In one aspect, the monomeric analogue is an INSL3 B-chain analogue modified from the sequence set forth in SEQ ID NO:7.

In a further aspect, the INSL3 analogue is constrained by a cross-link between a first amino acid within a range of positions 2 and 8 and a second amino acid within a range of positions 21 and 26 of the sequences set forth in SEQ ID NO:7.

In a particularly preferred form, the first and/or second amino acids are substituted with amino acids suitable for cross-linking.

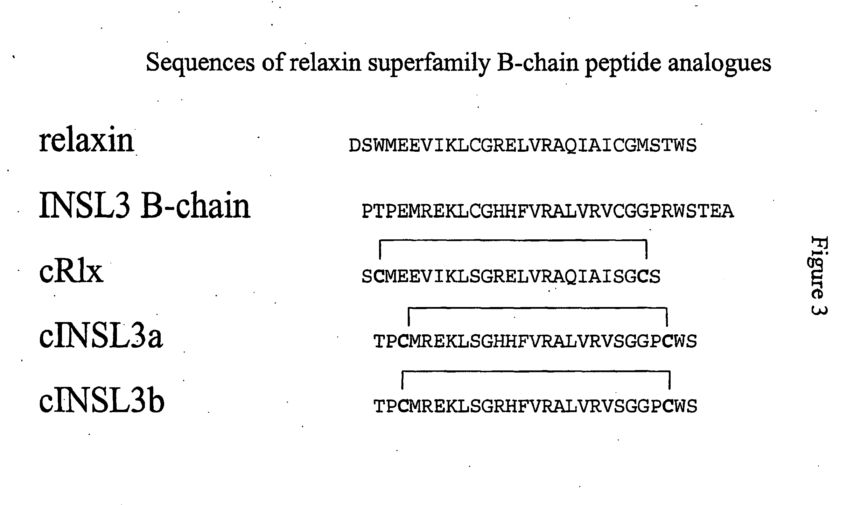

According to another aspect, the monomeric analogue is the INSL3 analogue designated cINSLa in Figure 3.

In a further aspect, the monomeric analogue is the INSL3 analogue designated cINSLb in Figure 3.

In yet another aspect, the monomeric analogue is a relaxin analogue modified from a relaxin-1, relaxin-2, or relaxin-3 B-chain sequence set forth in SEQ ID NOs: 1, 2 and 3, , respectively.

In a preferred form, the monomeric relaxin analogue is the relaxin analogue designated cRlx in Figure 3.

In a further aspect, the monomeric relaxin analogue is constrained by a cross-link between a first amino acid within a range of positions 2 and 8 and a second amino acid within a range of positions 21 and 26 of the sequences set forth in SEQ ID NO:2.

In yet a further form, one or more amino acids within the INSL3 or relaxin peptide analogue sequence, other than the cross-linked first and second amino acids, is optionally substituted to modify one or more biological activities of the monomeric analogue.

In a further preferred form, the monomeric analogue is an agonist, partial agonist, antagonist, partial antagonist or reverse antagonist at the relaxin superfamily receptor.

In another aspect, the analogues of the invention are conjugated to an A-chain of the relaxin superfamily protein.

In a further aspect, the monomeric analogues of the invention are conjugated to carrier molecules, such as labelling compounds, radioligands, radionucleotides, immunotherapeutic agents or other desirable chemical moiety.

In a further aspect, the invention seeks to provide a method of making a monomeric peptide analogue of a relaxin superfamily protein, the method comprising:
- providing a B-chain, receptor-binding subunit of a relaxin superfamily protein; and
- identifying a first and a second amino acid on respective, opposing strands of a turn and/or loop moiety in the receptor-binding subunit of the protein, with an alpha-helix or beta-strand carbon separation distance of less than six angstroms; and
- introducing a cross-link between the first and second amino acids;
wherein the cross-link constrains a three dimensional structure of at the B-chain, receptor-binding subunit.

In a particularly preferred form, the first and/or second amino acids are substituted with amino acids suitable for cross linking.

In one aspect, the method produces an INSL3 peptide analogue based on the sequence set forth in SEQ ID NO:7.

In a preferred form, the method produces the INSL3 analogue designated cINSLa in Figure 3.

In another preferred form, the method produces the INSL3 analogue designated cINSLb in Figure 3.

In a further aspect, the method produces a monomeric relaxin-1, relaxin-2 or relaxin-3 peptide analogue based on any of the sequences set forth in SEQ ID NOs:1, 2 and 3, respectively.

Preferably, the method produces a monomeric relaxin analogue designated cRlx in Figure 3.

In yet a further form, the method produces an INSL3 and/or relaxin analogue wherein one or more amino acids other than the cross-linked first and second amino acids, are optionally substituted to modify one or more biological activities of the peptide analogue.

In yet another aspect, there is provided a pharmaceutical composition including one or more of the monomeric analogues of the present invention, and pharmaceutically acceptable salts thereof.

Preferably, the pharmaceutical composition includes an INSL3 analogue or a relaxin analogue.

A method of treating, preventing or ameliorating the symptoms of a disorder in a patient, the disorder involving disregulation of activity of a relaxin superfamily protein member, the method comprising administering to the patient an effective amount of a peptide analogue of the invention.

In one aspect, the disorder is characterised by disregulation of relaxin and/or INSL3 expression or activity.

In another aspect, the invention provides a method of treating, preventing or ameliorating the symptoms of a relaxin or INSL3-mediated disorder in a patient, wherein the disorder is selected from the group consisting of hyperplastic disorders, neoplastic disorders, scleroderma, uncontrolled or abnormal collagen or fibronectin formation or breakdown, neurological disorders, angiogenic disorders, cardiovascular disorders, female reproductive disorders, conditions associated with pregnancy, renal disease, inflammatory bowel disease, Raynaud's disease, Raynaud's phenomenon, cryptorchidism, disregulation of spermatogenesis and reproductive development including descent of the gonads, the method comprising administering to the patient an effective amount of a peptide analogue of the invention.

In a further aspect, the invention contemplates the use of the analogues and/or pharmaceutical compositions of the present invention in the manufacture of a medicament for the treatment of a relaxin or INSL3-mediated disorder in a patient, the disorder selected from the group consisting of: hyperplastic disorders, neoplastic disorders, scleroderma, uncontrolled or abnormal collagen or fibronectin formation or breakdown, neurological disorders, angiogenic disorders, cardiovascular disorders, female reproductive disorders, conditions associated with pregnancy, renal disease, inflammatory bowel disease, Raynaud's disease, Raynaud's phenomenon, cryptorchidism, disregulation of spermatogenesis and reproductive development including descent of the gonads.

### Description of the Figures

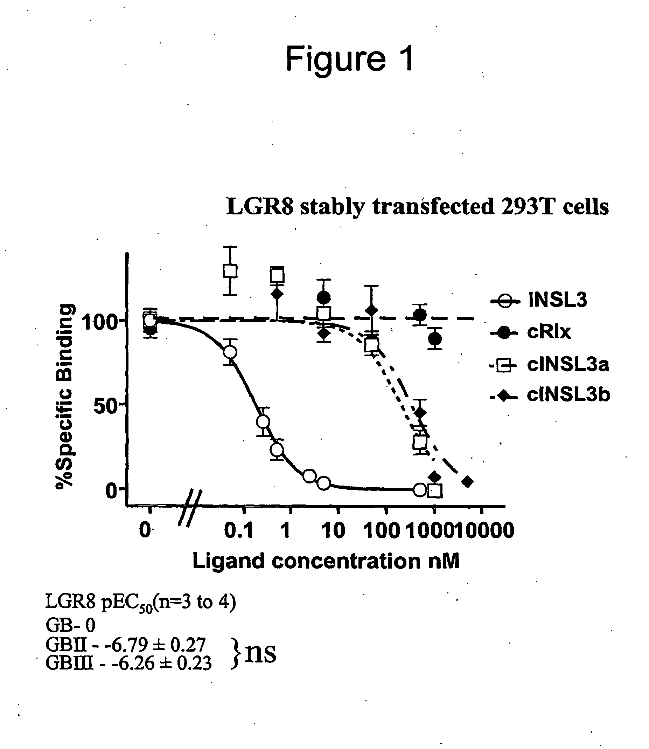

FIGURE 1: Schematic representation of binding of INSL3, cINSLa and cINSLb analogues to the LGR8 receptor.

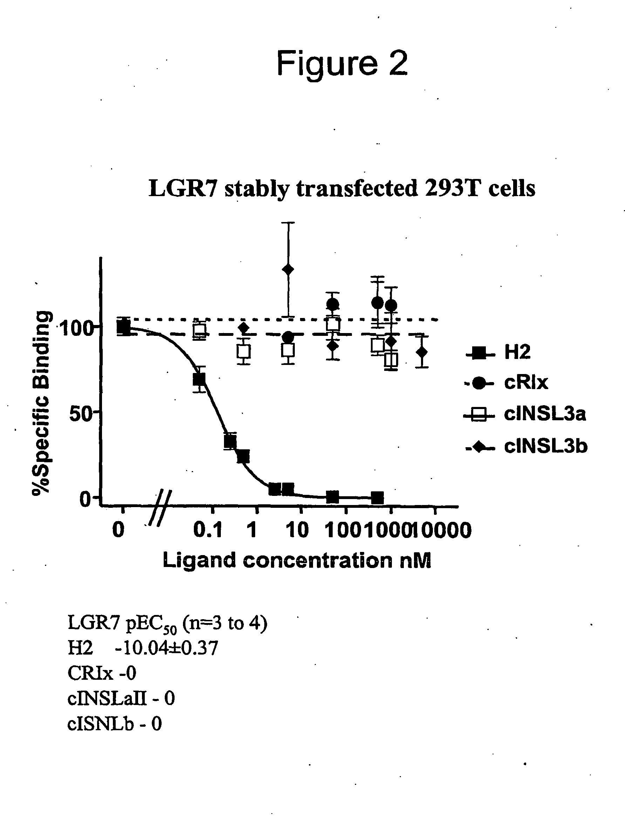

FIGURE 2: Schematic representation of binding of the relaxin-like or INSL3 analogues to the LGR7 receptor.
FIGURE 3: Sequences and constraints of the native relaxin and INSL3-B chains with exemplary analogues designed.

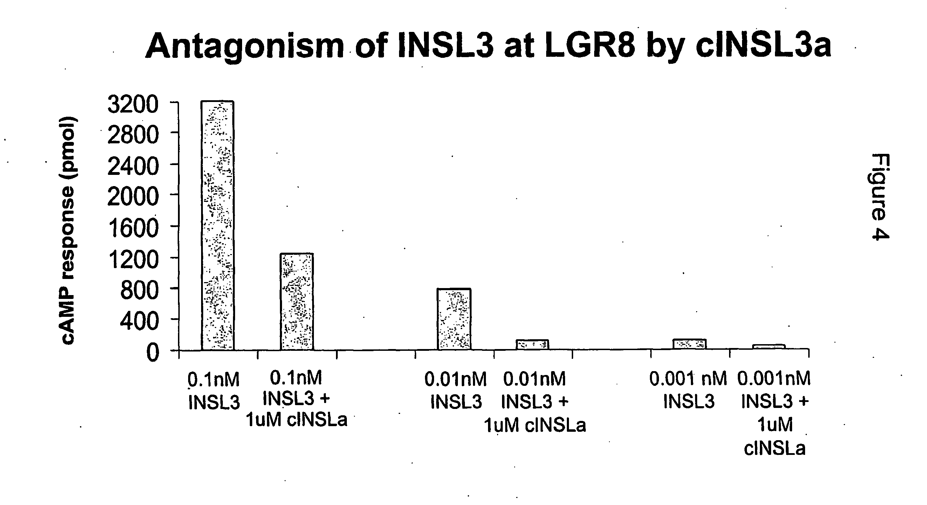

FIGURE 4: Schematic representation of antagonistic activity of cINSLa in the inhibition of the response of INSL3 (measured by cAMP response) to the native INSL3 receptor (LGR8).

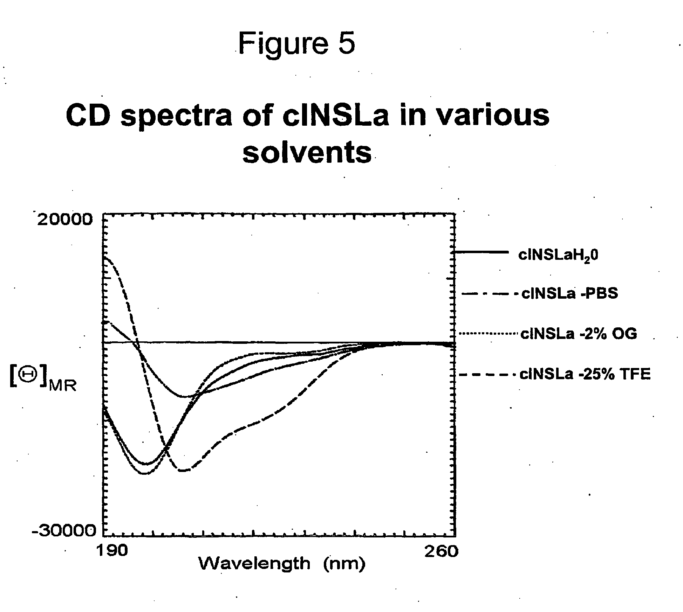

FIGURE 5: Schematic representation illustrating CD spectra of cINSLa showing significant alpha-helical content in water and phosphate buffered saline.

### Detailed Description

In its broadest form, the present invention relates to peptide analogues of members of the relaxin superfamily of proteins, the analogues having a binding activity at a relaxin protein superfamily receptor, methods of producing the analogues, and pharmaceutical compositions, methods of treatment, and uses of the peptide analogues.

In a first aspect, the analogue is a conformationally constrained, cyclic analogue of a B-chain of a relaxin superfamily member protein which binds to a biological target of a relaxin superfamily protein, and modulates an activity of the biological target, wherein the relaxin superfamily protein is selected from insulin, IGF-I, IGF-II, relaxin 1, relaxin 2, relaxin 3, INSL3, INSL4, INSL5 or INSL6.

The term "modulates" as used herein includes, but is not limited to, initiation of activity at a biological target of a relaxin superfamily protein, up- or down-regulation of activity at a biological target of a relaxin superfamily protein, or blockage of an activity at a biological target of a relaxin superfamily protein.

The biological targets of relaxin superfamily proteins include receptors, such as but not limited to, LGR7, LGR8, GPCR135, GPCR142, the insulin receptor, the IGF-I receptor, and the IGF-II receptor.

Accordingly, the analogue can also be a ligand at a relaxin superfamily receptor, wherein the analogue ligand can have an activity including, but not limited to, that of an agonist, antagonist, mixed agonist, reverse agonist, partial agonist, partial antagonist or reverse antagonist at the receptor.

The cyclic B-chain peptide analogues of the invention are produced by modification of a turn or loop moiety of the B-chain of the relaxin superfamily protein, the modification involving selection of at least a first and a second amino acid residue with an alpha-helix or beta-strand carbon separation distance of less than six Angstroms and cross-linking the first and second amino acids, wherein the cross-link conformationally constrains the analogue.

In one aspect, the analogue is an INSL3 B-chain analogue modified from the sequence set forth in SEQ ID N0:7.

In a further aspect, the INSL3 analogue is constrained by a cross-link between a first amino acid within a range of positions 2 and 8 and a second amino acid within a range of positions 21 and 26 of the sequences set forth in SEQ ID NO:7. Even more preferably, the INSL3 analogue is constrained by a cross-link between a first amino acid within a range of positions 2 and 8, including positions 2, 3, 4, 5, 6, 7, and 8, and a second amino acid within a range of positions 21 and 26, including 21, 22, 23, 24, 25 and 26, of the sequence set forth in SEQ ID NO:7.

In a particularly preferred aspect, the B-chain peptide analogue is the INSL3 analogue designated cINSLa in Figure, 3.

In yet another preferred form, the B-chain peptide analogue is the INSL3 analogue designated cINSLb in Figure 3.

In yet another aspect, the analogue is a relaxin analogue modified from a relaxin-1, relaxin-2, or relaxin-3 B-chain sequence set forth in SEQ ID NOs: 1, 2 and 3, respectively.

In a further aspect, the relaxin analogue is constrained by a cross-link between a first amino acid within a range of positions 2 and 8 and a second amino acid within a range of positions 21 and 26 of the sequences set forth in SEQ ID NO:2. Even more preferably, the relaxin analogue is constrained by a cross-link between a first amino acid within a range of positions 2 and 8, including positions 2, 3, 4, 5, 6, 7, and 8, and a second amino acid within a range of positions 21 and 26, including 21, 22, 23, 24, 25 and 26, of the sequence set forth in SEQ ID NO:2.

In a preferred form, the B-chain analogue is the relaxin analogue designated CRlx in Figure 3.

In a particularly preferred form, the first and second amino acids are substituted with amino acids suitable for cross linking.

Suitable chemical linking groups have 0 to 20 carbon atoms, 0 to 10 heteroatoms (N,O, S, P etc.), and can be straight chain or branched an contain saturated, unsaturated and/or aromatic rings, single and/or double bonds and chemical groups such as amide, ester, disulfide, thioether, ether, phosphate, amine and the like.

The amino-acid substitution and formation of a cross-link, which is used to conformationally constrain the B-chain can be obtained by several methods, including but not limited to:
(i) cyclizing the N-terminal amine with the C-terminal carboxyl acid function, either directly via an amide bond between the N-terminal nitrogen and C-terminal carbonyl, or indirectly via a spacer group, for example by condensation with an M-amino carboxylic acid;
(ii) cyclizing via the formation of a covalent bond between the side chains of two residues, such as an amide bond between a lysine residue and either an aspartic acid or glutamic acid residue, or a disulfide bond between two cysteine residues, or a thioether bond between a cysteine residue and anz-halogenated amino acid residue, either directly or via a spacer group as described in (i) above. The residues contributing the side chains may be derived from the B-chain sequence itself, or may be incorporated into or added on to the B-chain sequence for this purpose; and,
(iii) cyclizing via the formation of an amide bond between a side chain (for example of a lysine or aspartate residue) and either the C-terminal carboxyl or N-terminal amine, either directly or using a spacer group as described in (i) above. The residues contributing the side chains may be derived from the B-chain sequence itself, or may be incorporated into or added on to the B-chain sequence for this purpose.

Furthermore, the B-chain peptide analogue can be made in which one or more amino acid residues is replaced by its corresponding D-amino acid, substitutions or modifications are made to one or more amino acids in the sequence, peptide bonds can be replaced by a structure more resistant to metabolic degradation and different cyclizing constraints and dimerization groups can be incorporated.

With respect to compounds in which one or more amino acids is replaced by its corresponding D-amino acid, the skilled person is aware that retro-inverso amino acid sequences can be synthesized by standard methods; see, for example, Chorev and Goodman, Acc. Chem. Res., 26: 266-273,1993.

Olson et al., J. Med. Chem., 36: 3039-3049 1993 provides an example of replacing a peptide bond with a structure more resistant to metabolic degradation.

Analogues of the invention can also be made where individual amino acids are replaced by analogous structures, for example gem-diaminoalkyl groups or alkylmalonyl groups, with or without modified termini or alkyl, acyl or amine substitutions to modify their charge. The use of such alternative structures can provide significantly longer half-life in the body since they are more resistant to breakdown under physiological conditions.

Methods for combinatorial synthesis of peptide analogues and for screening of peptides and peptide analogs are well known in the art (see, for example, Gallop et al., J. Med Chem., 37: 1233-1251,1994). It is particularly contemplated that the analogues of the invention are useful as templates for design and synthesis of compounds of improved activity, stability and bioavailability.

Preferably where amino acid substitution is used, the substitution is conservative, i.e. an amino acid is replaced by one of similar size and with similar charge properties.

As used herein, the term "conservative substitution" denotes the replacement of an amino acid residue by another, biologically similar residue. Examples of conservative substitutions include the substitution of one hydrophobic residue such as isoleucine, valine, leucine, alanine, cysteine, glycine, phenylalanine, proline, tryptophan, tyrosine, norleucine or methionine for another, or the substitution of one polar residue for another, such as the substitution of arginine for lysine, glutamic acid for aspartic acid, or glutamine for asparagine, and the like. Neutral hydrophilic amino acids which can be substituted for one another include asparagine, glutamine, serine and threonine. The term "conservative substitution "also includes the use of a substituted amino acid in place of an unsubstituted parent amino acid.

As such, it should be understood that in the context of the present invention, a conservative substitution is recognized in the art as a substitution of one amino acid for another amino acid that has similar properties. Exemplary conservative substitutions are set out in the following Table A from WO 97/09433.

**TABLE A**

| **CONSERVATIVE SUBSTITUTIONS I** | |
|---|---|
| **Side Chain Characteristic** | **Amino Acid** |
| Aliphatic non-polar | G A P I L V |
| Polar - uncharged | C S T M N Q |
| Polar - charged | D E K R |
| Aromatic | H F W Y |
| Other | N Q D E |

Alternatively, conservative amino acids can be grouped as described in Lehninger, [Biochemistry, Second Edition; Worth Publishers, Inc. NY: NY(1975), pp. 71-77] as set out in the following Table B.

**TABLE B**

| **CONSERVATIVE SUBSTITUTIONS II** | |
|---|---|
| **Side Chain Characteristic** | **Amino Acid** |
| **Non-polar (hydrophobic)** | |
| A. Aliphatic: | A L I V P |
| B. Aromatic: | F W |
| C. Sutphur-containing: | M |
| D. Borderline: | G |
| **Uncharged-polar** | |
| A. Hydroxyl: | S T Y |
| B. Amides | N Q |
| C. Sulfhydryl: | C |
| D. Borderline: | G |
| Positively Charged (Basic): | K R H |
| Negatively Charged (Acidic): | D E |

Exemplary conservative substitutions are set out in the following Table C.

**TABLE C**

| **CONSERVATIVE SUBSTITUTIONS III** | |
|---|---|
| **Original Residue** | **Exemplary Substitution** |
| Ala (A) | Val, Leu, Ile |
| Arg (R) | Lys, Gln, Asn |
| Asn (N) | Gln, His, Lys, Arg |
| Asp (D) | Glu |
| Cys (C) | Ser |
| Gln (Q) | Asn |
| Glu (E) | Asp |
| His (H) | Asn, Gln, Lys, Arg |
| Ile (I) | Leu, Val, Met, Ala, Phe |
| Leu (L) | Ile, Val, Met, Ala, Phe |
| Lys (K) | Arg, Gln, Asn |
| Met (M) | Leu, Val, Ile, Ala |
| Pro (P) | Gly |
| Ser (S) | Thr |
| Thr (T) | Ser |
| Trp (W) | Tyr, Phe |
| Tyr (Y) | Trp; Phe, Thr, Ser |
| Val (V) | Ile, Leu, Met, Phe, Ala |

The cyclic peptide analogues of the invention can be modified, for instance, by glycosylation, amidation, carboxylation, or phosphorylation, or by the creation of acid addition salts, amides, esters, in particular C-terminal esters, and N-acyl derivatives of the peptides of the invention.

The peptide analogues also can be modified to create peptide derivatives by forming covalent or noncovalent complexes with other moieties. Covalently-bound complexes can be prepared by cross-linking the chemical moieties to functional groups on the side chains of amino acids comprising the peptides, or at the N-or C terminus.

The B-chain peptide analogues can be conjugated to an A-chain of a relaxin superfamily protein, and preferably, the A-chain of the relaxin superfamily protein to which they share B-chain analogy.

The B-chain peptide analogues can be conjugated to a reporter group, including, but not limited to a radiolabel, a fluorescent label, an enzyme (e.g., that catalyzes a colorimetric or fluorometric reaction), a substrate, a solid matrix, or a carrier (e. g., biotin or avidin).

In yet a further form, one or more amino acids within the peptide analogue sequence, other than the first and second amino acids, is optionally substituted to modify one or more biological activities of the analogue.

The term "biological activities" as used herein includes, but is not limited to, binding affinity for a biological target, effector activity at the biological target and *in vivo* stability.

In a further aspect, the invention seeks to provide a method of making a peptide analogue of a relaxin superfamily protein, the method comprising:
- providing a B-chain, receptor-binding subunit of a relaxin superfamily protein; and
- identifying a first and a second amino acid on respective, opposing strands of a turn and/or loop moiety in the receptor-binding subunit of the protein, with an alpha-helix or beta-strand carbon separation distance of less than six angstroms; and
- introducing a cross-link between the first and second amino acids;
wherein the cross-link constrains a three dimensional structure of at the B-chain, receptor-binding subunit.

In a particularly preferred form, the first and second amino acids are substituted with amino acids suitable for cross linking.

In one aspect, the method produces an INSL3 peptide analogue based on the sequence set forth in SEQ ID NO:7.

In a preferred form, the method produces an INSL3 analogue designated cRlx, cINSL3a or cINSL3b in Figure 3.

In a further aspect, the method produces a relaxin-1, relaxin-2 or relaxin-3 peptide analogue based on any of the sequences set forth in SEQ ID NOs:1, 2 and 3, respectively.

Preferably, the method produces a relaxin analogue designated cRlx in Figure 3.

In yet a further form, the method produces an INSL3 and/or relaxin analogue wherein one or more amino acids other than the cross-linked first and second amino acids, are optionally substituted to modify one or more biological activities of the peptide analogue.

One aspect of the invention utilises the ability of the B-chain peptide analogues of the invention to modulate an activity of a biological target of at least one relaxin superfamily protein member, that is, induce or modify an activity of at least one relaxin superfamily protein member, and in particular, relaxin or INSL3, which are mediated through the actions of the LGR7 or LGR8 receptors. The analogues can thus induce an activity of a relaxin superfamily member, in the absence of endogenous expression of the protein, or suppress or antagonise the activity of a protein endogenously expressed. Alternatively, the analogues of the invention can up-regulate the level of an existing biological activity of a relaxin superfamily member. Such biological activities include, but are not limited to, collagen degradation and remodelling, vascular dilation, growth and differentiation of the mammary gland, increased blood flow and organ perfusion, increasing heart rate, increasing coronory blood flow, increasing glomerular filtration rate and renal plasma flow, maturation of the gonads, promotion of cellular division, survival of sperm cells, development of ovarian follicles and oocyte maturation. As LGR8 is also expressed in the kidney, bone, peripheral blood leukoctyes, muscle, brain, thyroid, and uterus. Hence INSL3 analogues have potential actions on kidney and thyroid function, central actions on the brain, modulation of muscle function, effects on bone and blood cell function and reproductive actions on the uterus.

Accordingly, modification of any one or more of the above-mentioned biological activities of the relaxin superfamily proteins requires administration of an effective, biological-activity interfering amount of a B-chain analogue of the invention.

Clinical applications of the invention include, but are not limited to, hyperplastic disorders, neoplastic disorders, neurological disorders, angiogenic disorders, cardiovascular disorders, female reproductive disorders, conditions associated with pregnancy, renal disease, inflammatory bowel disease, Raynaud's disease, Raynaud's phenomenon, cryptorchidism, disregulation of spermatogenesis, male and female infertility and reproductive development including descent of the gonads.

In yet another aspect, there is provided a pharmaceutical composition including one or more of the analogues of the present invention, and pharmaceutically acceptable salts thereof.

Another aspect of the invention concerns the provision of a pharmaceutical composition comprising a B-chain analogue of the invention, and a pharmaceutically acceptable nontoxic salt thereof, and a pharmaceutically acceptable solid or liquid carrier or adjuvant. A preferred pharmaceutical composition will inhibit or interfere with a biological activity induced by at least one member of a relaxin superfamily protein: A particularly preferred pharmaceutical composition will inhibit the biological activity induced by relaxin and/or INSL3.

Examples of such a carrier or adjuvant include, but are not limited to, saline, buffered saline, Ringer's solution, mineral oil, talc, corn starch, gelatin, lactose, sucrose, microcrystalline cellulose, kaolin, mannitol, dicalcium phosphate, sodium chloride, alginic acid, dextrose, water, glycerol, ethanol, thickeners, stabilizers, suspending agents and combinations thereof. Such compositions may be in the form of solutions, suspensions, tablets, capsules, creams, salves, elixirs, syrups, wafers, ointments or other conventional forms.

The formulation is carried out to suit the mode of administration. Compositions comprising a peptide of the invention will contain from about 0. 1% to 90% by weight of the active compound(s), and most generally from about 10% to 30%.

The dose(s) and route of administration will depend upon the nature of the patient and condition to be treated, and will be at the discretion of the attending physician or veterinarian.

Suitable routes include oral, subcutaneous, intramuscular, intraperitoneal or intravenous injection, parenteral, topical application, implants etc. For example, an effective amount of a monomeric monocyclic peptide of the invention or a dimeric bicyclic peptide of the invention is administered to an organism in need thereof in a dose between about 0.1 and 1000µg/kg body weight.

The B-chain analogues of the invention may be used as therapeutic compositions either alone or in combination with other therapeutic agents.

Preferably, the pharmaceutical composition includes an INSL3 analogue or a relaxin analogue.

In one aspect, the disorder is characterised by disregulation of relaxin and/or INSL3 expression or activity.

As previously mentioned, it is understood that relaxin binds to at least two biological targets: LGR7 and LGR8, while INSL3 appears to mediate its biological effects via LGR8 only. Without being bound to any particular theory or mode of action, it is believed that an analogue with relaxin-like binding activity which exhibits affinity for LGR7 and LGR8 will modulate the effects of both relaxin and INSL3, given that INSL3 shares a common receptor with relaxin. Given the fact that LGR8 binds both relaxin and INSL3, it possible that many of relaxin's effects are modulated by INSL3 levels, and vice versa.

In another aspect, the invention provides a method of treating, preventing or ameliorating the symptoms of a relaxin or INSL3-mediated disorder in a patient, the disorder selected from the group consisting of hyperplastic disorders, neoplastic disorders, scleroderma, uncontrolled or abnormal collagen or fibronectin formation or breakdown, neurological disorders, angiogenic disorders, cardiovascular disorders, female reproductive disorders, conditions associated with pregnancy, renal disease, male and female infertility, inflammatory bowel disease, Raynaud's disease, Raynaud's phenomenon, cryptorchidism, disregulation of spermatogenesis and reproductive development including descent of the gonads, the method comprising administering to the patient an effective amount of a peptide of the invention.

As used herein, the term "conditions associated with pregnancy" includes, but is not limited to, conditions of fertilisation, pregnancy, parturition and lactation.

The term "relaxin or INSL3-mediated" as used herein refers to conditions where relaxin or INSL3 are involved or implicated in the development, maintenance or progression of the condition.

In a further aspect, the invention contemplates the use of the analogues and/or pharmaceutical compositions of the present invention in the manufacture of a medicament for the treatment of a relaxin or INSL3-mediated disorder in a patient, the disorder selected from the group consisting of: hyperplastic disorders, neoplastic disorders, scleroderma, neurological disorders, angiogenic disorders, cardiovascular disorders, female reproductive disorders, conditions associated with pregnancy, renal disease, male and female infertility, inflammatory bowel disease, Raynaud's disease, Raynaud's phenomenon, cryptorchidism, disregulation of spermatogenesis and reproductive development including descent of the gonads.

This invention will now be described with reference to the following, non-limiting examples.

### Example 1

### Methods and Materials.

### Molecular design of INSL3 analogues

Using SYBYL molecular modelling software (Tripos) on a Silicon Graphics 02 workstation, we designed a model of INSL3 B-chain from the X-ray crystal structure of human Gene 2 relaxin B-chain as a template (Eigenbrot et al., 1991, J Mol Biol 221: 15-21). After modifying the sequence to resemble that of INSL3, an energy minimisation using a Tripos forcefield with Gasteiger-Marsili charges was carried out. From the resulting energy minimised model, two residues were identified (Glu⁴ and Arg²⁶) to have Cβ atoms within 4Å of each other. These residues were then replaced with cysteine and a disulphide bond formed to give the cyclic peptide cINSLa.

A second cyclic analogue cINSLb was formed by changing His¹² to Arg, to mimic a relaxin-like binding motif of Arg-X-X-Arg-X-X-X-Val.

### Solid-phase peptide synthesis:

The synthesis of the INSL3 analogues were achieved using the continuous flow Fmoc-methodology as previously described (Dawson et al., J Peptide Res 53:542-547, 1999). The solid support was Fmoc-PAL PEG-PS (PerSeptive Biosystems, USA), and HBTUactivated Fmoc-amino acids were used throughout.. N^{α}-Fmoc deprotection was with 20% piperidine in DMF. All derivatives were purchased from Auspep (Melbourne, Australia). Cleavage of the peptides from the solid support and side chain deprotection was achieved by a 3 hour treatment with trifluoroacetic acid (TFA) in the presence of phenol, thioanisole, ethanedithiol and water (82.5/5/5/2.5/5, v/v). The crude peptides were subjected to reversed-phase high performance liquid chromatography (RP-HPLC) on a Vydac C18 column (Hesperia, USA) using a 1%/min gradient of CH3CN in 0.1% aqueous TFA. Peptides were then oxidised in a buffer containing 10% dimethylsulfoxide (DMSO) for 24 hours and the reaction monitored on HPLC and by mass spectrometry.

### Characterization:

The purity of the synthetic peptide was assessed by analytical RP-HPLC, and matrix-assisted laser desorption time of flight (MALDITOF) mass spectrometry using a Bruker Biflex instrument (Bremen, Germany) in the linear mode at 19.5 kV. Peptide quantitation was by amino acid analysis of a 24 hour acid hydrolyzate using a GBC instrument (Melbourne, Australia).

### Circular dichroism (CD) spectroscopy:

CD spectra were taken on a Jasco J-720 instrument at room temperature in a 1 mm pathlength cell, Doubly distilled water, 10 mM sodium phosphate buffer containing 120 mM NaCl pH 7.4, and spectroscopy grade trifluoroethanol (TFE) were used as solvents. The peptide concentrations were made to 1µM, determined by quantitative RP-HPLC and amino acid analysis (ref). Curves were smoothed by the algorithm provided by Jasco, Mean residue ellipticity ([θ]_{MR}) is expressed in degrees^{x}cm2/dmole by using the molecular mass. CD spectra evaluations were based on comparison with known peptide conformations (Otvos, L., Jr. 1996. from Neuropeptide protocols. Irvine, G. & Williams, C., Eds. pp 153-161. Humana Press, Totowa, NJ).

### Functional cAMP assays:

Human 293T cells stably transfected with human LGR7, or LGR8 were preincubated in the presence of 0.25mM 3-isobutyl-1-methyl xanthine (IBMX, Sigma) before various treatments for 30 minutes. At the end of the incubation cells were lysed for measurement of cAMP using a well-characterized cAMP ELISA (Satoko et al., J Biol Chem, 278:7855-7862). All experiments were repeated at least three times using cells from independent transfections, Results are plotted as pmol cAMP/10⁴ cells.

### Ligand binding assays:

The method for binding of 33P-labelled relaxin to 293T cells stably transfected with either LGR7 or LGR8 has been described previously (Satoko et al., J Biol Chem, 278:7855-7862). Data are expressed as mean ± SEM of % specific binding of triplicate determinations performed on a least three independent plates of transfected cells. Data were plotted using the one-site competition functions of the PRISM program (Graphpad Inc., San Diego, USA).

### Example 2

Sites for the incorporation cyclising constraints to create B chain analogues were determined by molecular modelling studies. In the case of relaxin, distances between β-carbon atoms in the B chain were measured in the X-ray crystal structure of relaxin [6RLX], using the molecular modelling program Sybyl (Tripos Inc St Louis MO). Residue 25 (Met) within the C chain helix and residue 3 (Trp) in the strand were found to have a β-carbon to β-carbon distance of 3.855 angstroms. These residues were thus deemed suitable for replacement by Cys and subsequent formation of a disulphide bond. This constrained two ends of the molecule in a similar fashion to the native protein, resulting in the cyclic relaxin B chain mimetic cRlx (Figure 3). Methods are as described in International Patent Applications Nos. WO 00/75176 and WO 01/52875.

In the case of INSL3 we constructed a model of the 3D structure of INSL3 by substituting the appropriate amino acids into the relaxin structure, and carrying out an energy minimisation of the resultant INSL3 B chain. We then identified pairs of residues as potential sites for the incorporation of a Cys to Cys constraint, including the pair Glu4 and Arg26, residues adjacent to those replaced in the relaxin analogue, giving the cyclic INSL3 mimetic cINSLA (Figure 3).

Following the biological characterisation of peptides cRlx and cINSLa (Figure 3), an additional compound was prepared incorporating the Cys to Cys constraint of peptide cINSLa.This compound, designated cINSLb, was a INSL3-based sequence, in which His12 form the INSL3 sequence was replaced by Arg in an attempt to obtain an analogue of the putative relaxin receptor binding cassette which if absent in INSL3.

The same approach is used to prepare insulin B chain analogues and other relaxin superfamily analogues.

**TABLE 1**

| **SEQ ID NO:** | **RELAXIN SUPERFAMILY B-CHAIN PEPTIDE/ANALOGUE** |
|---|---|
| 1 | relaxin-1 b-chain |
| 2 | relaxin-2 b-chain |
| 3 | relaxin 3 b-chain |
| 4 | insulin b-chain |
| 5 | IGF-I b-chain |
| 6 | IGF-2 b-chain |
| 7 | INSL3 b-chain |
| 8 | INSL4 b-chain |
| 9 | INSL5 b-chain |
| 10 | INSL6 b-chain |
| 11 | cyclic relaxin b-chain mimetic |
| 12 | INSL3 b-chain based peptide analogue 4 |
| 13 | INSL3 b-chain based peptide analogue 5 |
| 14 | INSL3 b-chain based peptide analogue 6 |
| 15 | INSL3 b-chain based peptide analogue 7 |
| 16 | relaxin-1 a-chain |
| 17 | relaxin-2 a-chain |
| 18 | relaxin-3 a-chain |
| 19 | insulin a-chain (comparative) |
| 20 | IGF-1 a-chain |
| 21 | IGF2 a-chain |
| 22 | INSL3 a-chain (Ley I-L/RLF) |
| 23 | INSL4 a-chain (placentin/EPIL) |
| 24 | INSL5 a-chain |
| 25 | INSL6 a-chain |

## Claims

1. A monomeric, cyclic peptide analogue of a B-chain of a relaxin superfamily member protein which binds to a biological target of the relaxin superfamily protein, and modulates an activity of the biological target, wherein the analogue is produced by modification of a turn or loop moiety of the B-chain of the relaxin superfamily protein, the modification involving selection of at least a first and a second amino acid residue with an alpha-helix or beta-strand carbon separation distance of less than six angstroms and cross-linking the first and second amino acids, wherein the cross-link conformationally constrains the analogue
wherein the relaxin superfamily protein is selected from IGF-I, IGF-II. relaxin 1, relaxin 2, relaxin 3, INSL3, INSL4, INSL5 and INSL6.

2. The analogue according to claim 1, wherein the biological target is selected from insulin receptors, IGFR-I. IGFR-II, LGR7 and LGR8.

3. The analogue according to any one of claims 1 to 2, wherein the analogue is an INSL3 B-chain analogue modified from a sequence set forth in SEQ ID NO:7.

4. The analogue according to claim 3, wherein the INSL3 analogue is constrained by a cross-fink between a first amino acid within a range of positions 2 and 8 and a second amino acid within a range of positions 21 and 26 of the sequences set forth in SEQ ID NO:7.

5. The analogue according to any one of claims 1 to 2, which is a relaxin analogue modified from a relaxin-1, relaxin-2, or relaxin-3 B-chain sequence set forth in SEQ ID NOs:1,2 and 3, respectively.

6. The analogue according to claim 5, wherein the relaxin analogue is constrained by a cross-link between a first amino acid within a range of positions 2 and 8 and a second amino acid within a range of positions 21 and 26 of the sequence set forth in SEQ ID NO:2.

7. The analogue according to any one of claims 1 to 6, wherein the first and/or second amino acids are substituted with alternative amino acids suitable for cross-linking.

8. The analogue according to claim 7 wherein at least one of the alternative amino acids is a cysteine residue.

9. The analogue according to claim 8 wherein both of the alternative amino acid residues are cysteine residues.

10. The analogue according to claim 9 wherein the analogue is cross-linked by oxidizing the cysteine residues to form a disulfide bond between the cysteine residues.

11. The analogue according to any one of claims 1 to 4 or 7 to 10, wherein the analogue is an INSL3 analogue with the sequence and structure:

12. The analogue according to any one of claims 1 to 4 or 7 to 10, wherein the analogue is an INSL3 analogue with the sequence and structure:

13. The analogue according to any one of claims 1 to 1, 2, 5, 6, or 7 to 10, wherein the analogue is a relaxin analogue with the sequence and structure:

14. The analogue according to any one of claims 1 to 13 wherein the biological target of the analogue is LGR7 and/or LOR8.

15. The analogue according to claim 14, wherein activity of the biological target is initiated, up-regulated, down-regulated or otherwise blocked.

16. The analogue of any one of claims 1 to 15, wherein the analogue is conjugated to an A-chain of a relaxin superfamily protein.

17. The analogue according to claim 16, wherein the A-chain of the relaxin superfamily protein is derived from the relaxin superfamily protein from which the B chain analogue is derived.

18. The analogue according to any one of claims 1 to 17, wherein the analogue is conjugated to a reporter group.

19. The analogue according to claim 18, wherein the reporter group is a radiolabel.

20. The analogue according to claim 18, wherein the reporter group is a fluorescent label.

21. The analogue according to claim 18, wherein the reporter group is an enzyme.

22. The analogue according to claim 18, wherein the reporter group is a carrier.

23. A method of making a peptide analogue of a relaxin superfamily protein, the method comprising:
- providing a B-chain, receptor-binding subunit of a relaxin superfamily protein; and
- identifying a first and a second amino acid on respective, opposing strands of a turn and/or loop moiety in the receptor-binding subunit of the protein, with an alpha-helix or beta-strand carbon separation distance of less than six angstroms; and
- introducing a cross-link between the first and second amino acids;
wherein the cross-link conformationally constrains a three dimensional structure of at the B-chain, receptor-binding subunit.

24. The method according to claim 23, wherein the first and/or second amino acids are substituted with amino acids suitable for cross-linking.

25. The method according to claim 23 or 24, wherein the method produces an INSL3 peptide analogue derived from the sequence set forth in SEQ ID NO:7, with the first amino acid within a range of residues 2 and 8 and the second amino acid within a range of residues 21 and 26.

26. The method according to claim 23 or 24, wherein the method produces a relaxin peptide analogue derived from the sequence set forth in any one of SEQ ID NOS:1 to 3, with the first amino acid within a range of residues 2 and 8 and the second amino acid within a range of residues 21 and 26.

27. The method according to any one of claims 23 to 26, wherein the method produces an INSL3 or relaxin analogue wherein one or more amino acids other than the cross-linked first and second amino acids are substituted to modify one or more biological activities of the peptide analogue.

28. A pharmaceutical composition including one or more analogues as claimed in any one of claims 1 to 22.

29. The pharmaceutical composition according to claim 28, further comprising at least one pharmaceutically acceptable carrier or diluent.

30. Use of a peptide analogue according to any one of claims 1 to 22 for the manufacture of a medicament for treating, preventing or ameliorating the symptoms of a disorder associated with deregulation of activity of a relaxin superfamily protein member,

31. A peptide analogue according to any one of claims 1 to 22 for treating, preventing or ameliorating the symptoms of a disorder associated with deregulation of activity of a relaxin superfamily protein member

32. The use according to claim 30 or peptide analogue according to claim 31, wherein the disorder is mediated by relaxin or INSL3

33. The use or peptide analogue according to claim 32, wherein the disorder selected from the group consisting of hyperplastic disorders, scleroderma, uncontrolled or abnormal collagen or fibronectin formation or breakdown, neoplastic disorders, neurological disorders, angiogenic disorders, cardiovascular disorders, female reproductive disorders, conditions associated with pregnancy, renal disease, inflammatory bowel disease, Raynaud's disease, Raynaud's phenomenon, cryptorchidism, male or female infertility, disregulation of spermatogenesis and reproductive development including descent of the gonads.

34. The use or peptide analogue according to claim 33, wherein the disorder is a female reproductive disorder.

35. The use or peptide analogue according to claim 33, wherein the disorder is a pregnancy-related disorder.

36. The use or peptide analogue according to claim 33, wherein the disorder involves abnormal reproductive developments

37. The use or peptide analogue according to claim 33, wherein the disorder is a cardiovascular disorder

38. The use or peptide analogue according to claim 33, wherein the disorder is an angiogenic disorder.

39. The use or peptide analogue according to claim 33, wherein the disorder is a neoplastic disorder or a hyperplastic disorder

40. The use or peptide analogue according to claim 39,wherein the neoplastic or hyperplastic disorder involves the thyroid gland.

41. The use or peptide analogue according to claim 33, wherein the disorder is scleroderma.

42. The use or peptide analogue according to claim 33, wherein the disorder is renal disease.

43. The use or peptide analogue according to claim 33, wherein the disorder involves uncontrolled or abnormal collagen or fibronectin formation or breakdown

44. The use or peptide analogue according to claim 33, wherein the disorder is male or female infertility.

## Patentansprüche

1. Ein monomeres Cyclopeptid-Analogon einer B-Kette eines zur Relaxin-Superfamilie gehörenden Proteins, das sich mit einem biologischen Ziel des Proteins der Relaxin-Superfamilie verbindet und eine Aktivität des biologischen Ziels moduliert, wobei das Analogon durch Modifikation einer Windungs- oder Schleifenkomponente der B-Kette des Proteins der Relaxin-Superfamilie hergestellt wird, wobei die Modifikation die Auswahl von mindestens einem ersten und einem zweiten Aminosäurerest mit einem Alpha-Helix- oder Beta-Strang-Kohlenstoff-Trennungsabstand von weniger als sechs Ångström und das Queiveinetzen der ersten und zweiten Aminosäure involviert, wobei die Quervernetzung in konformer Weise das Analogen beschränkt, wobei das Protein der Relaxin-Superfamilie ausgewählt wird aus IGF-I, IGF-II, Relaxin-1, Relaxin-2, Relaxin-3, INSL3, INSL4, INSL5 und INSL6

2. Das Analogon gemäß Anspruch 1, wobei das biologische Ziel ausgewählt wird aus Insulinrezeptoren, IGFR-I, IGFR-II, LGR7 und LGR8

3. Das Analogon gemäß einem der Ansprüche 1 bis 2, wobei das Analogon ein INSL3-B-Ketten-Analogon ist, das aus einer in SEQ-ID Nr. 7 dargelegten Sequenz modifiziert wird

4. Das Analogon gemäß Anspruch 3, wobei das INSL3-Analogon durch eine Quervernetzung zwischen einer ersten Aminosäure innerhalb eines Bereichs der Positionen 2 und 8 und einer zweiten Aminosäure innerhalb eines Bereichs der Positionen 21 und 26 der in SEQ-ID Nr. 7 dargelegten Sequenzen beschränkt wird.

5. Das Analogon gemäß einem der Ansprüche 1 bis 2, das ein Relaxin-Analogon ist, das aus einer in SEQ-ID Nr. 1, 2 und 3 dargelegten Relaxin-1-, Relaxin-2- bzw Relaxin-3-B-Ketten-Sequenz modifiziert wird

6. Das Analogon gemäß Anspruch 5, wobei das Relaxin-Analogon durch eine Quervernetzung zwischen einer ersten Aminosäure innerhalb eines Bereichs der Positionen 2 und 8 und einer zweiten Aminosäure innerhalb eines Bereichs der Positionen 21 und 26 der in SEQ-ID Nr. 2 dargelegten Sequenz beschränkt wird.

7. Das Analogon gemäß einem der Ansprüche 1 bis 6, wobei die erste und/oder zweite Aminosäure durch alternative Aminosäuren ersetzt werden, die sich für die Quervernetzung eignen.

8. Das Analogon gemäß Anspruch 7, wobei mindestens eine der alternativen Aminosäuren ein Cysteinrest ist.

9. Das Analogon gemäß Anspruch 8, wobei beide alternative Aminosäurereste Cysteinreste sind.

10. Das Analogon gemäß Anspruch 9, wobei das Analogon durch Oxidieren der Cysteinreste zur Bildung einer Disulfidbindung zwischen den Cysteinresten quervernetzt wird.

11. Das Analogon gemäß einem der Ansprüche 1 bis 4 oder 7 bis 10, wobei das Analogon ein FNSL3-Analogon mit dieser Sequenz und Struktur ist:

12. Das Analogon gemäß einem der Ansprüche 1 bis 4 oder 7 bis 10, wobei das Analogon ein INSL3-Analogon mit dieser Sequenz und Struktur ist:

13. Das Analogon gemäß einem der Ansprüche 1 bis 2, 5, 6 oder 7 bis 10, wobei das Analogon ein Relaxin-Analogon mit dieser Sequenz und Struktur ist:

14. Das Analogon gemäß einem der Ansprüche 1 bis 13, wobei das biologische Ziel des Analogons LGR7 und/oder LGR8 ist

15. Das Analogon gemäß Anspruch 14, wobei die Aktivität des biologischen Ziels initiiert, hochgesteuert, heruntergesteuert oder in sonstiger Weise blockiert wird.

16. Das Analogon gemäß einem der Ansprüche 1 bis 15, wobei das Analogon mit einer A-Kette eines Proteins der Relaxin-Superfamilie konjugiert wird

17. Das Analogon gemäß Anspruch 16, wobei die A-Kette des Proteins der Relaxin-Superfamilie von dem Protein der Relaxin-Superfamilie abgeleitet wird, von dem das B-Ketten-Analogon abgeleitet wird.

18. Das Analogon gemäß einem der Ansprüche 1 bis 17, wobei das Analogon mit einer Reportergruppe konjugiert wird

19. Das Analogon gemäß Anspruch 18, wobei die Reportergruppe eine radioaktive Markierung ist

20. Das Analogon gemäß Anspruch 18, wobei die Reportergruppe eine fluoreszierende Markierung ist.

21. Das Analogon gemäß Anspruch 18, wobei die Reportergruppe ein Enzym ist.

22. Das Analogon gemäß Anspruch 18, wobei die Reportergruppe ein Träger ist.

23. Ein Verfahren zum Herstellen eines Peptidanalogons eines Proteins der Relaxin-Superfamilie, wobei das Verfahren umfasst:
Bereitstellen einer Rezeptoren bindenden B-Ketten-Untereinheit eines Proteins der Relaxin-Superfamilie; und
Bestimmen einer ersten und einer zweiten Aminosäure an jeweils entgegengesetzten Strängen einer Windungs- und/oder Schleifenkomponente in der Rezeptoren bindenden Untereinheit des Proteins mit einem Alpha-Helix- oder Beta-Strang-Kohlcnstoff-Trennungsabstand von weniger als sechs Ångström; und
das Einführen einer Quervernetzung zwischen der ersten und zweiten Aminosäure;
wobei die Quervernetzung in konformer Weise eine dreidimensionale Struktur an der Rezeptoren bindenden B-Ketten-Untereinheit beschränkt

24. Das Analogon gemäß Anspruch 23, wobei die erste und/oder zweite Aminosäure durch Aminosäuren ersetzt werden, die sich für die Quervernetzung eignen

25. Das Verfahren gemäß Anspruch 23 oder 24, wobei bei dem Verfahren ein INSL3-Peptidanalogon hergestellt wird, das von der in SEQ-ID Nr, 7 dargelegten Sequenz abgeleitet ist, mit der ersten Aminosäure innerhalb eines Bereichs der Reste 2 und 8 und der zweiten Aminosäure innerhalb eines Bereichs der Reste 21 und 26.

26. Das Verfahren gemäß Anspruch 23 oder 24, wobei bei dem Verfahren ein Relaxin-Peptidanalogon hergestellt wird, das von der in einer der SEQ-ID-Nummem 1 bis 3 dargelegten Sequenz abgeleitet ist, mit der ersten Aminosäure innerhalb eines Bereichs der Reste 2 und 8 und der zweiten Aminosäure innerhalb eines Bereichs der Reste 21 und 26

27. Das Verfahren gemäß einem der Ansprüche 23 bis 26, wobei bei dem Verfahren ein INSL3- oder Relaxin-Analogon hergestellt wird, wobei eine oder mehrere Aminosäure außer der quervernetzten ersten und zweiten Aminosäure ersetzt weiden, um eine oder mehrere biologische Aktivitäten des Peptidanalogons zu modifizieren.

28. Eine pharmazeutische Zusammensetzung, die ein oder mehrere Analoga gemäß einem der Ansprüche 1 bis 22 enthält

29. Die pharmazeutische Zusammensetzung gemäß Anspruch 28, die darüber hinaus mindestens ein pharmazeutisch annehmbares Träger- oder Verdünnungsmittel umfasst.

30. Die Verwendung eines Peptidanalogons gemäß einem der Ansprüche 1 bis 22 für die Herstellung eines Medikaments zur Behandlung, Vorbeugung oder Verbesserung der Symptome bei einer Erkrankung in Zusammenhang mit der Deregulierung von Aktivitäten eines zur Relaxin-Superfamilie gehörenden Proteins.

31. Ein Peptidanalogon gemäß einem der Ansprüche 1 bis 22 zur Behandlung, Vorbeugung oder Verbesserung der Symptome bei einer Erkrankung in Zusammenhang mit der Deregulierung von Aktivitäten eines zur Relaxin-Superfamilie gehörenden Proteins

32. Die Verwendung gemäß Anspruch 30 oder das Peptidanalogon gemäß Anspruch 31, wobei die Erkrankung durch Relaxin oder INSL3 mittelbar bewirkt wird.

33. Die Verwendung oder das Peptidanalogon gemäß Anspruch 32, wobei die Erkrankung ausgewählt wird aus der Gruppe, die besteht aus hyperplastischen Erkrankungen, Sklerodermie, unkontrollierter oder abnormer Bildung oder unkontrollicitem oder abnormem Abbau von Collagen oder Fibronectin, neoplastischen Erkrankungen, neurologischen Erkrankungen, angiogenen Erkrankungen, kardiovaskulären Erkrankungen, Erkrankungen des weiblichen Fortpflanzungssystems, Leiden im Zusammenhang mit der Schwangerschaft, Nierenleiden, chronisch-entzündlichen Darmcrkrankungen, der Raynaud-Krankheit, dem vasospastischen Phänomen, dem Kryptorchismus, der Unfruchtbarkeit beim Mann oder bei der Frau, Regulationsstörungen der Spermatogenese sowie der Entwicklung des Fortpflanzungssystems einschließlich des Gonadendeszensus

34. Die Verwendung oder das Peptidanalogon gemäß Anspruch 33, wobei die Erkrankung eine Erkrankung des weiblichen Fortpflanzungssystems ist

35. Die Verwendung oder das Peptidanalogon gemäß Anspruch 33, wobei die Erkrankung eine schwangerschaftsbezogene Erkrankung ist.

36. Die Verwendung oder das Peptidanalogon gemäß Anspruch 33, wobei die Erkrankung abnorme Entwicklungen des Fortpflanzungssystems involviert.

37. Die Verwendung oder das Peptidanalogon gemäß Anspruch 33, wobei die Erkrankung eine kardiovaskuläre Erkrankung ist

38. Die Verwendung oder das Peptidanalogon gemäß Anspruch 33, wobei die Erkrankung eine angiogene Erkrankung ist.

39. Die Verwendung oder das Peptidanalogon gemäß Anspruch 33, wobei die Erkrankung eine neoplastische Erkrankung oder eine hyperplastische Erkrankung ist

40. Die Verwendung oder das Peptidanalogon gemäß Anspruch 39, wobei die neoplastische Erkrankung oder die hyperplastische Erkrankung die Schilddrüse involviert,

41. Die Verwendung oder das Peptidanalogon gemäß Anspruch 33, wobei die Erkrankung Sklerodermie ist

42. Die Verwendung oder das Peptidanalogon gemäß Anspruch 33, wobei die Erkrankung eine Neubildungskrankheit ist

43. Die Verwendung oder das Peptidanalogon gemäß Anspruch 33, wobei die Erkrankung die unkontrollierte oder abnorme Bildung oder den unkontrollierten oder abnormen Abbau von Collagen oder Fibronectin involviert.

44. Die Verwendung oder das Peptidanalogon gemäß Anspruch 33, wobei die Erkrankung die Unfruchtbarkeit beim Mann oder bei der Frau ist.

## Revendications

1. Un analogue peptidique cyclique monomère d'une chaîne B d'une protéine membre de la superfamille des relaxines qui se lie à une cible biologique de la protéine de la superfamille des relaxines et module une activité de la cible biologique, où l'analogue est produit par modification d'un fragment de boucle ou de coude de la chaîne B de la protéine de la superfamille des relaxines, la modification impliquant la sélection d'au moins un premier et un deuxième résidus d'acide aminé avec une hélice alpha ou une distance de séparation carbone brin bêta de moins de six angströms et une réticulation des premier et deuxième acides aminés, où la réticulation contraint conformationnellement l'analogue,
où la protéine de la superfamille des relaxines est sélectionnée parmi IGF-I, IGF-II, relaxine 1, relaxine 2, relaxine 3, INSL3, INSL4, INSL5 et INSL6

2. L'analogue selon la Revendication 1 où la cible biologique est sélectionnée parmi les récepteurs d'insuline, IGFR-I, IGFR-II, LGR7 et LGR8.

3. L'analogue selon la Revendication 1 ou 2, où l'analogue est un analogue de chaîne B INSL3 modifié à partir d'une séquence définie dans SEQ ID N°7.

4. L'analogue selon la Revendication 3, où l'analogue INSL33 est contraint par une réticulation entre un premier acide aminé à l'intérieur d'une plage des positions 2 et 8 et un deuxième acide aminé à l'intérieur d'une plage des positions 21 et 26 des séquences définies dans SEQ ID N°7

5. L'analogue selon l'une quelconque des Revendications 1 à 2, qui est un analogue de relaxine modifié à partir d'une séquence de chaîne B de relaxine-1 , relaxine-2 ou relaxine-3 définie respectivement dans SEQ ID N°1, 2 et 3

6. L'analogue selon la Revendication 5, où l'analogue de relaxine est contraint par une réticulation entre un premier acide aminé à l'intérieur d'une plage des positions 2 et 8 et un deuxième acide aminé à l'intérieur d'une plage des positions 21 et 26 de la séquence définie dans SEQ ID N°2 ,

7. L'analogue selon l'une quelconque des Revendications 1 à 6, où les premier et/ou deuxième acides aminés sont remplacés par d'autres acides aminés qui conviennent à une réticulation.

8. L'analogue selon la Revendication 7 où au moins un des autres acides aminés est un résidu cystéine.

9. L'analogue selon la Revendication 8 où les deux autres résidus d'acide aminé sont des résidus cystéines

10. L'analogue selon la Revendication 9 où l'analogue est réticulé par oxydation des résidus cystéines de façon à former une liaison disulfure entre les résidus cystéines

11. L'analogue selon l'une quelconque des Revendications 1 à 4 ou 7 à 10 où l'analogue est un analogue INSL3 présentant la séquence et la structure :

12. L'analogue selon l'une quelconque des Revendications 1 à 4 ou 7 à 10 où l'analogue est un analogue INSL3 présentant la séquence et la structure :

13. L'analogue selon l'une quelconque des Revendications 1 à 2, 5, 6 ou 7 à 10 où l'analogue est un analogue de relaxine présentant la séquence et la structure :

14. L'analogue selon l'une quelconque des Revendications 1 à 13 où la cible biologique de l'analogue est LGR7 et/ou LGR8

15. L'analogue selon la Revendication 14, où l'activité de la cible biologique est démarrée, régulée à la hausse, régulée à la baisse ou autrement bloquée

16. L'analogue selon l'une quelconque des Revendications 1 à 15, où l'analogue est conjugué à une chaîne A d'une protéine de la superfamille des relaxines

17. L'analogue selon la Revendication 16, où la chaîne A de la protéine de la superfamille des relaxines est dérivée de la protéine de la superfamille des relaxines de laquelle l'analogue de chaîne B est dérivé.

18. L'analogue selon l'une quelconque des Revendications 1 à 17, où l'analogue est conjugué à un groupe rapporteur.

19. L'analogue selon la Revendication 18, où le groupe rapporteur est un radiomarqueur.

20. L'analogue selon la Revendication 18, où le groupe rapporteur est un marqueur fluorescent

21. L'analogue selon la Revendication 18, où le groupe rapporteur est une enzyme

22. L'analogue selon la Revendication 19, où le groupe rapporteur est un vecteur

23. Un procédé de fabrication d'un analogue peptidique d'une protéine de la superfamille des relaxines, le procédé comprenant :
- la fourniture d'une sous-unité de liaison au récepteur de chaîne B d'une protéine de la superfamille des relaxines, et
- l'identification d'un premier et d'un deuxième acide aminé sur des brins opposés respectifs d'un fragment de boucle et/ou de coude dans la sous-unité de liaison au récepteur de la protéine avec une hélice alpha ou une distance de séparation carbone brin bêta de moins de six angströms, et
- l'introduction d'une réticulation entre les premier et deuxième acides aminés, où la réticulation contraint conformationnellement une structure tridimensionnelle de la sous-unité de liaison au récepteur de chaîne B

24. Le procédé selon la Revendication 23, où les premier et/ou deuxième acides aminés sont remplacés par des acides aminés qui conviennent à une réticulation

25. Le procédé selon la Revendication 23 ou 24, où le procédé produit un analogue peptidique INSL3 dérivé de la séquence définie dans SEQ ID N°7 avec le premier acide aminé à l'intérieur d'une plage de résidus 2 et 8 et le deuxième acide aminé à l'intérieur d'une plage de résidus 21 et 26

26. Le procédé selon la Revendication 23 ou 24, où le procédé produit un analogue peptidique de relaxine dérivé de la séquence définie dans SEQ ID N°1 à 3 avec le premier acide aminé à l'intérieur d'une plage de résidus 2 et 8 et le deuxième acide aminé à l'intérieur d'une plage de résidus 21 et 26.

27. Le procédé selon l'une quelconque des Revendications 23 à 26, où le procédé produit un analogue de relaxine ou INSL3 où un ou plusieurs acides aminés autres que les premier et deuxième acides aminés réticulés sont remplacés de façon à modifier une ou plusieurs activités biologiques de l'analogue peptidique.

28. Une composition pharmaceutique contenant un ou plusieurs analogues tels que définis dans l'une quelconque des Revendications 1 à 22.

29. La composition pharmaceutique selon la Revendication 28, comprenant en outre au moins un vecteur ou diluant pharmaceutiquement acceptable

30. L'utilisation d'un analogue peptidique selon l'une quelconque des Revendications 1 à 22 pour la fabrication d'un médicament destiné à traiter, prévenir ou améliorer les symptômes d'un trouble associé au dérèglement de l'activité d'une protéine membre de la superfamille des relaxines

31. Un analogue peptidique selon l'une quelconque des Revendications 1 à 22 destiné à traiter, prévenir ou améliorer les symptômes d'un trouble associé au dérèglement de l'activité d'une protéine membre de la superfamille des relaxines.

32. L'utilisation selon la Revendication 30 ou un analogue peptidique selon la Revendication 31, où le trouble est médié par une relaxine ou INSL3

33. L'utilisation ou un analogue peptidique selon la Revendication 32, où le trouble est sélectionné dans le groupe se composant de troubles hyperplasiques, sclérodermie, formation ou dissociation de fibronectine ou de collagène anormale ou non contrôlée, troubles néoplasiques, troubles neurologiques, troubles angiogéniques, troubles cardiovasculaires, troubles de la reproduction chez la femme, états associés à la grossesse, maladies rénales, infection abdominale inflammatoire, maladie de Raynaud, syndrome de Raynaud, cryptorchidie, infertilité chez l'homme ou la femme, dérèglement de la spermatogenèse et développement reproductif incluant la descente des glandes génitales.

34. L'utilisation ou un analogue peptidique selon la Revendication 33, où le trouble est un trouble de la reproduction chez la femme.

35. L'utilisation ou un analogue peptidique selon la Revendication 33, où le trouble est un trouble lié à la grossesse.

36. L'utilisation ou un analogue peptidique selon la Revendication 33, où le trouble implique des développements reproductifs anormaux

37. L'utilisation ou un analogue peptidique selon la Revendication 33, où le trouble est un trouble cardiovasculaire.

38. L'utilisation ou un analogue peptidique selon la Revendication 33, où le trouble est un trouble angiogénique.

39. L'utilisation ou un analogue peptidique selon la Revendication 33, où le trouble est un trouble néoplasique ou un trouble hyperplasique.

40. L'utilisation ou un analogue peptidique selon la Revendication 39, où le trouble hyperplasique ou néoplasique implique la glande thyroïde

41. L'utilisation ou un analogue peptidique selon la Revendication 33, où le trouble est une sclérodermie.

42. L'utilisation ou un analogue peptidique selon la Revendication 33, où le trouble est une maladie de renouvellement.

43. L'utilisation ou un analogue peptidique selon la Revendication 33, où le trouble implique une formation ou une dissociation de fibronectine ou de collagène anormale ou non contrôlée.

44. L'utilisation ou un analogue peptidique selon la Revendication 33, où le trouble est une infertilité chez l'homme ou la femme.


### SEQUENCE LISTING

<110> Howard Florey Institute of Experimental Physiology and Medicine The University of Melbourne
<120> Relaxin Superfamily Peptide Analogues
<130> 12262441/PAS/SET
<150> AU 2003903124
   <151> 2003-06-20
<160> 25
<170> PatentIn version 3.1
<210> 1
   <211> 28
   <212> PRT
   <213> Homo sapiens
<400> 1
   Lys Trp Lys Asp Asp Val Ile Lys Leu Cys Gly Arg Glu Leu Val Arg
   1               5                   10                  15
   Ala Gln Ile Ala Ile Cys Gly Met Ser Thr Trp Ser
               20                  25
<210> 2
   <211> 29
   <212> PRT
   <213> Homo sapiens
<400> 2
   Asp Ser Trp Met Glu Glu Val Ile Lys Leu Cys Gly Arg Glu Leu Val
   1               5                   10                  15
   Arg Ala Gln Ile Ala Ile Cys Gly Met Ser Thr Trp Ser
               20                  25
<210> 3
   <211> 27
   <212> PRT
   <213> Homo sapiens
<400> 3
   Arg Ala Ala Pro Tyr Gly Val Arg Leu Cys Gly Arg Glu Phe Ile Arg
   1               5                   10                  15
   Ala Val Ile Phe Thr Cys Gly Gly Ser Arg Trp
               20                  25
<210> 4
   <211> 30
   <212> PRT
   <213> Homo sapiens
<400> 4
   Phe Val Asn Gln His Leu Cys Gly Ser His Leu Val Glu Ala Leu Tyr
   1 5 10 15
   Leu Val Cys Gly Glu Arg Gly Phe Phe Tyr Thr Pro Lys Thr
   20 25 30
<210> 5
   <211> 29
   <212> PRT
   <213> Homo sapiens
<400> 5
   Gly Pro Glu Thr Leu Cys Gly Ala Glu Leu Val Asp Ala Leu Gln Phe
   1 5 10 15
   Val Cys Gly Asp Arg Gly Phe Tyr Phe Asn Lys Pro Thr
   20 25
<210> 6
   <211> 31
   <212> PRT
   <213> Homo sapiens
<400> 6
   Tyr Arg Pro Ser Glu Thr Leu Cys Gly Gly Glu Leu Val Asp Thr Leu
   1 5 10 15
   Gln Phe Val Cys Gly Asp Arg Gly Phe Tyr Phe Ser Arg Pro Ala
   20 25 30
<210> 7
   <211> 31
   <212> PRT
   <213> Homo sapiens
<400> 7
   Pro Thr Pro Glu Met Arg Glu Lys Leu Cys Gly His His Phe Val Arg
   1 5 10 15
   Ala Leu Val Arg Val Cys Gly Gly Pro Arg Trp Ser Thr Glu Ala
   20 25 30
<210> 8
   <211> 33
   <212> PRT
   <213> Homo sapiens
<400> 8
   Glu Ser Leu Ala Ala Glu Leu Arg Gly Cys Gly Pro Arg Phe Gly Lys
   1 5 10 15
   His Leu Leu Ser Tyr Cys Pro Met Pro Glu Lys Thr Phe Thr Thr Thr
   20 25 30
   Pro
<210> 9
   <211> 33
   <212> PRT
   <213> Homo sapiens
<400> 9
   Val Arg Ser Lys Glu Ser Val Arg Leu Cys Gly Leu Glu Tyr Ile Arg
   1 5 10 15
   Thr Val Ile Tyr Ile Cys Ala Ser Ser Arg Trp Arg Arg His Leu Glu
   20 25 30
   Gly
<210> 10
   <211> 33
   <212> PRT
   <213> Homo sapiens
<400> 10
   Ser Asp Ile Ser Ser Ala Arg Lys Leu Cys Gly Arg Tyr Leu Val Lys
   1               5                   10                  15
   Glu Ile Glu Lys Leu Cys Gly His Ala Asn Trp Ser Gln Phe Arg Phe
               20                  25                  30
   Glu
<210> 11
   <211> 25
   <212> PRT
   <213> artificial sequence
<220>
   <223> relaxin-2 b-chain based peptide analogue
<400> 11
   Ser Cys Met Glu Glu Val Ile Lys Leu Ser Gly Arg Glu Leu Val Arg
   1               5                   10                  15
   Ala Gln Ile Ala Ile Ser Gly Cys Ser
               20                  25
<210> 12
   <211> 27
   <212> PRT
   <213> artificial sequence
<220>
   <223> INSL-3 b-chain based peptide analogue 4
<400> 12
   Thr Pro Cys Met Arg Glu Lys Leu Ser Gly His His Phe Val Arg Ala
   1 5 10 15
   Leu Val Arg Val Ser Gly Gly Pro Cys Trp Ser
   20 25
<210> 13
   <211> 27
   <212> PRT
   <213> artificial sequence
<220>
   <223> INSL-3 b-chain based peptide analogue 5
<400> 13
   Thr Pro Cys Met Arg Glu Lys Leu Ser Gly Arg His Phe Val Arg Ala
   1 5 10 15
   Leu Val Arg Val Ser Gly Gly Pro Cys Trp Ser
   20 25
<210> 14
   <211> 27
   <212> PRT
   <213> artificial sequence
<220>
   <223>INSL-3 b-chain based peptide analogue 6
<400> 14
   Thr Pro Cys Met Arg Glu Lys Leu Ser Gly Arg Glu Leu Val Arg Ala
   1 5 10 15
   Gln Val Ile Ala Ile Gly Gly Pro Cys Trp Ser
   20 25
<210> 15
   <211> 27
   <212> PRT
   <213> artificial sequence
<220>
   <223> INSL-3 b-chain based peptide analogue 7
<400> 15
   Thr Cys Glu Met Arg Glu Lys Leu Ser Gly His His Phe Val Arg Ala
   1 5 10 15
   Leu Val Arg Val Ser Gly Gly Cys Arg Trp Ser
   20 25
<210> 16
   <211> 24
   <212> PRT
   <213> Homo sapiens
<400> 16
   Arg Pro Tyr Val Ala Leu Phe Glu Lys Cys Cys Leu Ile Gly Cys Thr
   1 5 10 15
   Lys Arg Ser Leu Ala Lys Tyr Cys
   20
<210> 17
   <211> 24
   <212> PRT
   <213> Homo sapiens
<400> 17
   Gln Leu Tyr Ser Ala Leu Ala Asn Lys Cys Cys His Val Gly Cys Thr
   1               5                   10                  15
   Lys Arg Ser Leu Ala Arg Phe Cys
               20
<210> 18
   <211> 24
   <212> PRT
   <213> Homo sapiens
<400> 18
   Asp Val Leu Ala Gly Leu Ser Ser Ser Cys Cys Lys Trp Gly Cys Ser
   1               5                   10                  15
   Lys Ser Glu Ile Ser Ser Leu Cys
               20
<210> 19
   <211> 26
   <212> PRT
   <213> Homo sapiens
<400> 19
   Ser Leu Gln Lys Arg Gly Ile Val Glu Gln Cys Cys Thr Ser Ile Cys
   1               5                   10                  15
   Ser Leu Tyr Gln Leu Glu Asn Tyr Cys Asn
               20                  25
<210> 20
   <211> 25
   <212> PRT
   <213> Homo sapiens
<400> 20
   Ala Pro Gln Thr Gly Ile Val Asp Glu Cys Cys Phe Arg Ser Cys Asp
   1 5 10 15
   Leu Arg Arg Leu Glu Met Tyr Cys Ala
   20 25
<210> 21
   <211> 25
   <212> PRT
   <213> Homo sapiens
<400> 21
   Arg Arg Ser Arg Gly Ile Val Glu Glu Cys Cys Phe Arg Ser Cys Asp
   1 5 10 15
   Leu Ala Leu Leu Glu Thr Leu Cys Ala
   20 25
<210> 22
   <211> 26
   <212> PRT
   <213> Homo sapiens
<400> 22
   Ala Ala Ala Thr Asn Pro Ala Arg Tyr Cys Cys Leu Ser Gly Cys Thr
   1 5 10 15
   Gln Gln Asp Leu Leu Thr Leu Cys Pro Tyr
   20 25
<210> 23
   <211> 25
   <212> PRT
   <213> Homo sapiens
<400> 23 <210> 24
   <211> 24
   <212> PRT
   <213> Homo sapiens
<400> 24
<210> 25
   <211> 24
   <212> PRT
   <213> Homo sapiens
<400> 25
   Arg Lys Arg Arg Gly Tyr Ser Glu Lys Cys Cys Leu Thr Gly Cys Thr
   1               5                   10                  15
   Lys Glu Glu Leu Ser Ile Ala Cys
               20